# EUROPEAN PATENT APPLICATION

(11) **EP 2 657 892 A1**
(43) Date of publication of application: **30.10.2013**
(21) Application number: 11836700.2
(22) Date of filing: 06.05.2011
(51) Int. Cl.: G06Q 10/00, G06Q 50/22

(54) **CLINICAL INFORMATION SYSTEM**

(30) Priority: 29.10.2010 RU 2010144144
(71) Applicant: Obschestvo s Ogranichennoy Otvetstvennostiu «Pravovoe Soprovojdenie Bisnesa», Moscow 119146 (RU)
(72) Inventor: GLOTKO, Vladimir Leonidovich, Dolgoprudniy 119146 (RU); RUDNEV, Alexandr Sergeevich, Moscow 117602 (RU); KARAULANOV, Anton Alexandrovich, Moscow 117420 (RU); MODENOV, Evgeniy Alexandrovich, Moscow 124617 (RU)
(74) Representative: Zimmermann & Partner
(86) International application number: PCT/RU2011/000313
(87) International publication number: WO 2012/057649

(57) **Abstract**

The invention relates to medicine and can be used as a clinical information system. The clinical information system comprises computerized stations for an attending doctor, which stations are equipped with input/output devices and are connected by a network to a message checker with a subsystem for inputting source information about each patient, for the attending doctor to differentiate between clinical and instrumental-laboratory data to establish a diagnosis, the above-mentioned subsystem for inputting source information communicates via an exchange busbar with an information carrier subsystem which has the function of reproducing information reference material on the display of the computerized work station of the attending doctor, which reference material corresponds to the type of information input into the subsystem for inputting source information about each patient, and the above-mentioned subsystem for inputting source information is connected via a general busbar to a general medical database and to an intellectually analytical and statistical information processing unit, which communicate with each other in an information exchange mode, and to a unit for forming medical accounts.

## Description

The invention relates to medicine and can be used in outpatient clinics and hospitals as a clinical information system comprising a smart help system that enables the differentiation of clinical, instrumentation, and laboratory data in order to make a diagnosis. The computer-assisted clinical system is designed to support medical decision making in diagnosing, choosing treatment options, and prescribing drugs.

### Prior Art

Medical establishments have lists of mandatory and optional examinations and procedures and physiotherapy treatments for patients. There are recommendations of the RF Public Health Ministry and standards for efficient examination and medicamental treatment of patients with various profiles. The selection of procedures for diagnosis and treatment of various pathologies is made by the attending physician based on the above-listed aids and personal hands-on experience of the attending physician and head of department. This causes medical facilities of the public health service to incur a variety of labour and other costs, depending on the option chosen per assistance scheme per patient, regarding changes made to the treatment regimen in connection with complications or comorbidity identified in the course of treatment, which is difficult to factor in when determining actual staff workloads and quality of assistance provided. Also, the introduction of new technologies and advanced equipment and their full use is also a factor to be considered in improving the quality of medical services provided.

Previous attempts to solve the problems of the public health service regarding timely information support or introduction of statistical programmes involved various forms of automation. Some of those attempts were in the form of an automated medical hotline. Other attempts were aimed at providing physicians with computer-assisted tools for patient examination. Those solutions involved static procedures or algorithms based on the requirement of fast diagnosis and choice-of-treatment option using a computer and with due regard to the database and the algorithm of its operation. The objective in this context was for the computer rather than the physician to make a diagnosis automatically, using standard input to describe the patient. Such solutions use the algorithm of interviewing the patient to gather necessary and sufficient information about the patient to the extent required by at least one of the computer's medical diagnosis scenarios. Such a scheme for building a computer based on a set of medical diagnosis scenarios does not require the doctor's involvement until the stage of patient data input and in the final analysis shapes the "convenient" mentality that everything can be made to fit one of the scenarios. This system of medical assistance does not provide for the involvement of the doctor either as an expert or primary diagnostician. Also, such a system assumes that an effective method has been developed for specialists to contribute their medical expertise in the format of scenarios that constantly populate the database of the computer. Scenarios must use dynamic structures for fast and effective patient diagnosis.

An example of such a computer-assisted system is the solution described in WO 98/02836, G06F19/00, issued 22.01.1998. The document describes a computer-assisted diagnosis system that over a period of time repeatedly asks questions to be answered by the patient, with answers establishing symptoms that change over time. Every symptom that is established adds weight to the disease, develops one or more synergistic weights based on the symptoms established (with the development of synergistic weights in this context including the establishment of a synergistic symptom, accumulation of the weights of the established symptoms, and synergetic weights for the disease) and the chosen set of the symptoms established that emerges in the predetermined sequence over time adding additional diagnostic weight to the disease, and determines whether the resulting cumulative weight for the disease reaches or passes the threshold, based on which it makes and pronounces the diagnosis.

In generating a set of input information about the patient and a set of symptoms, the automatic medical diagnosis system produces a road map for treating the disease, which the physician chooses from the options in a list of diseases that exhibit to a greater or lesser extent the features of the course of the disease that are related to the established list of symptoms that is based on patient source data. In this case, the doctor switches from one regime to the next (from one scenario to another) to select a likely diagnosis based on the identification of a syndrome.

This technical solution is assumed as prior art nearest the claimed system.

In the prior art system, diagnosis is made based on a disease diagnosis input chart, which provides for the identification of the object of the first disease which is related to the set of symptom objects of the first disease in which at least one symptom object of the first disease has an actual symptom weight, and the identification of the object of the second disease which [object] is related to the set of symptom objects of the second disease, with at least one symptom object of the second disease matching at least one symptom object of the first disease and having an alternative symptom weight. In such a procedure for symptom evaluation, diagnosis is made based on the choice of a disease for which this symptom in other diseases has minimum value.

This prior art system is designed to have terminals for communicating with the system (PC) that are equipped with information input/output devices and are linked by a remote access network with a communication controller and a subsystem for input of source information about each patient that comprises a module for the input of basic details of the patient, a module for the input of information from the medical history with the patient's complaints, and a module for the input of details of the attending physician's clinical tests, the subsystem being linked to the database containing the diagnosis scenarios and the corresponding appropriate treatment options and methods and specifying the pharmaceutical component required.

A disadvantage of this system as a clinical information system or a doctor's smart help system for diagnosis and treatment development is that it is built on the principle of categorizing diseases, symptoms, and questions into a set of interlinked structures of diseases, symptoms, and questions, such as objects or lists, so that the structures can be accessed to set up a dialogue with the patient. In the prior art system, differentiation (or choice) is built on the principle of elimination of concepts (clinical features, symptoms). Also, the patient's subjective complaints are reviewed and ascertained in terms of objectivity by a test program running in an automated mode rather than by a medical specialist. The patient may be unaware of the exact location of a given organ; complaints about it can start a specific test run in the wrong direction, with the system doing a search using the input provided, the result being wasted time and a false positive. The disadvantage is that clinical differentiation assigns weights and weight synergy to a given symptom, which leads to the evaluation of an averaged condition rather than that of the patient (the weight setting and weight synergy of a given symptom are preset in the program as some kind of fixed factor). This approach to the evaluation of the patient's condition is highly likely to result in a diagnosis that is consistent with symptom weight (assigned based on questionnaires for the sole reason that this is the strongest manifestation in the patient and psychologically suppresses less pronounced forms of the occurrence of other symptoms) but in actual fact this can be an indirect symptom caused by another process. A biased description of the first pronounced symptoms that the system received from the patient during the first polling stage of operation results in the wrong disease scenario retrieved from the database and an incorrect diagnosis made. The system has no functionality to monitor the progress of the disease and the results of the treatment, which makes it impossible for the system to make adjustments by updating symptom weight or altering the scenario.

### Invention Disclosure

This invention is aimed at improving the performance of the system and the reliability of results by implementing the process of differentiation and analysis of recorded clinical data (indicators of the clinical condition of the patient; these are records of complaints and examination by a specialist, as well as laboratory and instrumentation data) using clinical matrices in order to provide advisory and specialized support for the medical specialist's decision making in the diagnostic and treatment process for each individual patient.

The above-mentioned technical result is achieved through the configuration of the clinical information system characterized by the attending physicians' computerized workstations, which are equipped with input/output devices and linked by a network with a communication controller and a subsystem for input of source information about each patient, the controller and subsystem being comprised of a module for input of basic details of the patient, a module for the input of information from the medical history of the patient's complaints, a module for the input of details of the clinical tests done by the attending physician, a module for the input of information based on the results of instrumentation and laboratory tests, a module for the input of details of treatment options and methods, and a module for the recording of the diagnosis made by the attending physician to differentiate among clinical, instrumentation, and laboratory data by the attending physician in order to make a diagnosis. The said subsystem for source data input is linked by a data bus with an information support subsystem that displays information and reference material on the attending physician's monitor consistent with the format of the data entered into the subsystem for input of source data of each patient, and comprises a module with information on general reference sources, this module being linked to the module for the input of the patient's basic details; a module with information on the groups of diseases that contain matches for the patient's complaints, which is linked to the module for the input of information from the medical history with the patient's complaints; a module with information on clinical tests, which is linked to the module for the input of details of clinical tests; a module with information on instrumentation and laboratory tests, which is linked to the module for the input of information based on the results of instrumentation and laboratory tests; a module with information on the forms of standards of generic and specific treatment, which is linked to the module for the input of details of treatment options and methods; and a module with information on the diagnoses made, which is linked to the module for the recording of the diagnosis made by the attending physician, said subsystem for the input of source data is linked via the common bus with the common medical database, the module of smart analysis and statistical processing of information, the module of computer-aided information processing, which are interlinked in the data exchange mode, and the medical reporting module, with the module of smart analysis and statistical processing of information having been designed to enable the implementation of the function of mathematical and analytical treatment of the patient's details in real time based on the particulars of the patient's clinical condition, the dynamics of disease progress and the response to the treatment, the module computer-aided information processing designed to enable the implementation of the function of provision of pharmacological data and supporting real-time comparison and analysis of data on various pharmacological groups of medicinal drugs between themselves, and comprising data bus-interlinked database on pharmacological products and a database on drug compatibility and interaction.

The above features are essential and are key in forming a fixed set of essential features that is sufficient to produce the required technical result.

### Brief Description of Drawings

This invention is illustrated with an embodiment, which, however, is not the only one possible, but graphically demonstrates the feasibility of achieving the required technical result.

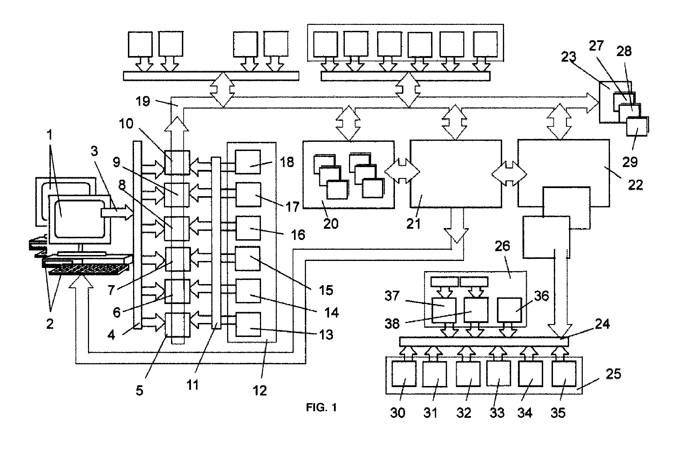

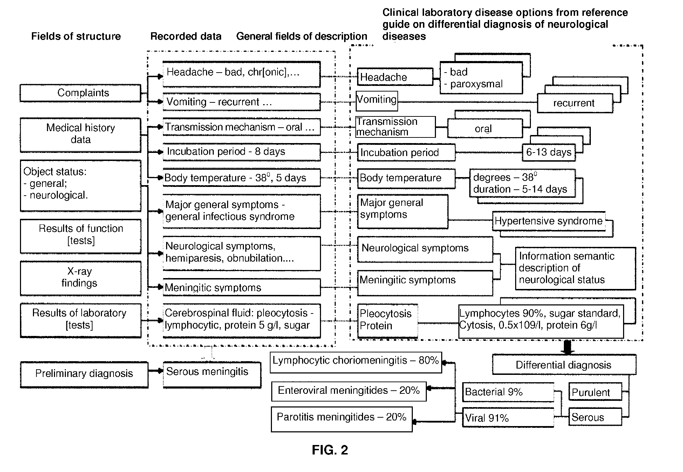

Fig. 1 is a block diagram of the clinical information system, and Fig. 2 is a flow chart of differential diagnosis using the system in Fig. 1.

### Preferred Embodiment of Invention

This invention involves a computer-based reference system for information support of diagnostic and treatment process by recording, formalization, accumulation, exchange, analysis, and processing of individual and group medical data over time.

The online wizard system is designed to support routine clinical activities of physicians of various profiles making differential diagnosis of various diseases. The system enables differential diagnosis in terms of either certain clinical criteria or group clinical data (i.e., symptoms and syndromes). The system uses a software-based method for differential diagnosis by group of multiple features and combination thereof in common conjugate fields.

Authority for scientifically valid medical decisions in everyday clinical practice of medical specialists is provided by the result of differential diagnosis of diseases. Normally, an experienced physician makes a diagnosis, and even a differential diagnosis, directly following review of the medical history and examination of the patient. Differential diagnosis is a thought process that becomes virtually subconscious after the acquisition of certain skills and experience. Because of the brevity of the process, the process pattern is quite difficult to identify but is very important to do so in order to reduce the incidence of errors in diagnosis, to teach the methods of differential diagnosis, and to develop software-based methods for comparison of identified pathological changes using computer technology.

Differential diagnosis is predicated on the recognition of symptoms based on careful monitoring of the clinical picture of the patient and on faithful recording. All of the above comprises the stage of collection and entry into the database of general and specific medical data and clinical and instrumentation data for each specific patient. The next step is review and assessment of the data, which is an attempt to ascertain the value of individual symptoms, phenomena, subjective complaints, results of diagnostic tests, and provocative tests and their interplay.

Data recording and assessment is followed by differentiation, recognition of aetiologic, pathogenic, or symptomatic manifestations, and of known categories, and selection of those from among them which are most likely to occur. Physicians would find it much easier if the entire process could be completed using the laws of classical logic. Unfortunately, this is impossible. If diagnostics afforded the identification and categorization of phenomena as in mathematics and in other exact sciences, then differential diagnosis would not be needed at all, with everything readily lending itself to differentiation and identification.

Differentiation requires diagnostic algorithms and categories under which a given case can be classified. The process of differential diagnosis consists precisely in classifying, based on the available data, the set of symptoms observed under the disease categories that encompass it as fully as possible. It is therefore necessary to identify and establish the category of disease and to make a diagnosis that would explain the presence of all symptoms in evidence in this case. This is the reason why diseases are divided accordingly based on certain common patterns.

An important feature of differential analysis of medical information obtained is the application of the comparable analysis method to identical kinds of information. Differential analysis using multiple features identifies diagnostic criteria for underpinning a differential diagnosis.

For the convenience of differentiation using a medical database, classification has been developed for dividing diseases into groups, subgroups, subsubgroups, etc., all the way to specific units, i.e. diseases (a one-size-fits-all method developed for entry of clinical and other data (matrix), including a functionality matrix with all groups of diseases, for example, heart diseases, pneumonia, and peripheral nervous system disorders, but they all are part of a single matrix). In classical logic, the classification criterion is always an important or essential feature. In practice, however, it is most often the most useful parameter that is chosen as the criterion. In classical logic, classification is carried on to the end using one and the same criterion. In practical differential diagnosis, classification into specific subgroups can be done using different criteria for reasons of expediency.

Where used in practice, classification must in any event be comprehensive; the subgroups must encompass all variations encountered. Classification that divides all meningitides based on a cytogram of cerebrospinal fluid and leucocytosis of blood should not be used because there are many variations in the course of this disease where the test result of cerebrospinal fluid and leucocytosis of blood differs little if at all. Classification errors can be avoided by using procedures for comparison, collation, and identification of matches of multiple indicators and features. These aspects of differential analysis of incoming medical data are incorporated into the design of the proposed computer-based reference system for information support of diagnostic and treatment processes.

As per this invention, the computer-based reference system for information support of diagnostic and treatment process (Fig. 1) contains computerized workstations 1 of the attending physician, equipped with input/output devices 2 and linked by network 3 with communication controller 4 with a subsystem for the input of source data of each patient, which comprises module 5 for the input of the patient's basic details, module 6 for the input of information from the medical history with the patient's complaints, module 7 for the input of details of the clinical tests done by the attending physician on the patient, module 8 for the input of information based on the results of instrumentation and laboratory tests, module 9 for the input of details of treatment options and methods, and module 10 for the recording of the diagnosis made by the attending physician.

In this context, the said subsystem for the input of source data (modules 5 through 10) through data bus 11 is linked to information support subsystem 12, which is designed to display information and reference material on the monitor of computerized workstation 1 of the attending physician. This material is consistent with the format of the data entered into the subsystem for input of source data of each patient. Information support subsystem 12 comprises module 13 with information on general reference sources, which is linked to module 5 for the input of the patient's basic details; module 14 with information on the groups of diseases that contain matches for the patient's complaints, which is linked to module 6 for the input of information from the medical history with the patient's complaints; module 15 with information on clinical tests, which is linked to module 7 for the input of details of clinical tests; module 16 with information on instrumentation and laboratory tests, which is linked to module 8 for the input of information based on the results of instrumentation and laboratory tests; module 17 with information on the forms of standards of generic and specific treatment, which is linked to module 9 for the input of details of treatment options and methods; and module 18 with information on the diagnoses made, which is linked to module 10 for the recording of the diagnosis made by the attending physician.

The source data input subsystem is linked via common bus 19 to common medical database 20, module 21 of smart analysis and statistical processing of information, and module 22 of computer-aided information processing, which are interlinked in the data exchange mode, and medical reporting module 23.

Module 21 of smart analysis and statistical processing of information is engineered to enable the implementation of the function of mathematical and analytical treatment of the patient's details in real time based on the particulars of the patient's clinical condition, dynamics of disease progress and response to the treatment. Module 22 of computer-aided information processing is engineered to enable the implementation of the function of provision of pharmacological data and the implementation of real-time comparison and analysis of data on various pharmacological groups of medicinal drugs between themselves, and comprises pharmacological product database 25 and drug compatibility and interaction database 26, which is interlinked by data bus 24.

The computer-based reference system for information support of diagnostic and treatment process according to Fig. 1 uses the method of comparison and collation of descriptions of clinical features and the test scores obtained in a subgroup of a group of diseases. The next stage is the computation of criteria for (not percentages but criteria of exact, prevalent, likely, negative) matching between the identified features and the variations in the course of this subgroup of diseases in the system's medical database. Variations of clinical manifestations and the results of studies of the diseases being differentiated are stored in the database of medical reference sources, which is formed using the scientific literature available. The database of medical reference sources is being added to and updated on a regular basis.

The following criteria have been selected: exact (T) (inherent, pathognomonic, obligatory); prevalent ( 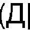 [D]) (very frequent, nearly always present); likely (B[V]) (frequent, sensitive); negation (O) (contradictory, incompatible, antagonist indicating the impossibility of presence of this feature (symptom) in a given disease).

The method of single conjugate fields is used to collate the parameters of several features recorded by medical specialists in the relevant sections of the database. In that way, differentiation between several distinct diseases is conducted in terms of incidence of identified features against those of the variants in a single group of diseases.

For example, a neurologist examines a patient suspected of having meningitis (G03 as per ICD-10). The information obtained is entered into the system in the appropriate fields: complaints (distinctive nature of headache, vomiting), medical history (epidemic aspects, transmission mechanisms, incubation period, temperature), objective status (general and neurological examination - prevailing symptoms and syndromes, meningeal and encephalitic symptoms (the system is predicated on semantics in the description of symptoms and syndromes, i.e., all clinical features), results of blood and cerebrospinal fluid tests, and results of serological and virological tests. The number of conjugate fields is determined by the user, i.e., the medical specialist. Fig. 2 depicts a flow chart of differential diagnosis using the online wizard system. The recording of information obtained is followed by differential diagnosis in terms of appropriate fields (chosen at discretion) using the section of differential diagnosis of neurological diseases. Medical specialists use online processing of information entered into the system and collate and compare the data gathered and received from the patient with the available options for the course of diseases in this subgroup (takes place automatically) in the guide on differential diagnosis of neurological diseases (meningitides of enteroviral aetiology, parotitis aetiology, lymphocytic choriomeningitis, meningitis form of poliomyelitis, etc). The result is returned upon submission of preliminary differential diagnoses, with their likely and prevalent manifestations in this clinical case, and is automatically updated upon entry of additional data identified. Differentiation is conducted as follows: the module of recording (entry) of information about the patient has sections and fields identical for the purposes of collation to the sections and fields in the *difdiagmat* [differential diagnosis material], i.e., they are logically conjugated.

Differentiation is conducted by subsystem 12; the result of differential-diagnostic analysis is presented, conveniently, [in] the form of a graph, where each identified disease is assigned a rating of prevalence and likelihood [of] presence in the specific patient. This model enables broadband, multiple, and multilevel differentiation systems, which makes it possible to factor in and identify the rarest forms of disease evolution and specific and generic forms of pathological process manifestations. Differentiation can also be undertaken with the patient unconscious, which is not unlikely in first aid.

Therefore, a distinctive feature of the developed smart online help system is computer-assisted search and context-specific (semantic) support for the term entered in recording, differential collation and support of clinical or other data:
- it issues general and specific symptoms and syndromes for the diagnosis provided (pre-installed updatable catalogue with the option of ongoing addition);
- it issues the most prevalent indicators of laboratory and instrumentation tests for the diagnosis provided (pre-installed updatable catalogue with the option of ongoing addition by customer's staff);
- it issues a plan for standard examination of the patient under the diagnosis provided (elements of the reference guide/templates are generated by the medical treatment facility on its own, or a framework reference guide is produced, which is then managed by the staff);
- it issues a plan for standard/generic treatment of the patient under the diagnosis provided (elements of the reference guide/templates are generated by the medical treatment facility on its own, or a framework reference guide is produced, which is then managed by the staff);
- it issues a plan for standard/generic sanatorium and spa treatment under the diagnosis provided (generated as above)[;] it automatically issues general contraindications for the treatment options under the diagnosis provided;
- it provides computer-assisted analysis of drug interaction and contraindications for the medicinal (drug) treatment provided;
- it provides computer-assisted analysis of the match between prescribed treatments (drugs) and the diagnosis provided and of the compatibility of treatments (drugs).

It enables the management and key staff of medical facilities to accomplish the following tasks:
- obtaining of accurate and full information about disease incidence in an organization or area;
- fast procurement of clinical and other information on each specific patient;
- continuous monitoring of diagnostic and treatment activities of medical officers and units;
- independent medical statistical analysis of any medical unit;
- real-time monitoring of the quality of the medical care provided for a specific patient and subordinate subdivisions;
- conducting computer-assisted comparative and collative analyses of the activities of different medical units over any given period of time; and
- operational report generation.

The components of subsystems (modules) interoperate using the linked-field algorithm, whereby each feature entered is correlated with a set of associated or respondent features, the selection from which is subject to a probability scheme in which every additional feature entered narrows the field of associated or respondent features related to the first feature entered. The method of single conjugate fields enables quantitative differentiation of several distinct diseases based on the incidence of identified features against those of the variants in a single group of diseases.

Medical reporting module 23 includes medical record generation module 27, medical record generation module 28, and module 29 for the generation of other medical documentation, whose electronic versions are put into common medical database 20.

Common medical database 20 is used as storage for all obtained medical and other details of the patient, personal identification details, medical history and a chronological sequence of the patient's complaints, for the gathering of medical history data, statistical and analytical processing of returned results, standard description by medical specialists of clinical data in textual and digital formats obtained during the examination of the patient - the identified feature (symptom) in one line, for analytical and comparable analysis study in terms of significance (weighted contribution) of each symptom to the formation of the syndrome and diagnosis, findings obtained by hardware facilities in any form, as well as an opinion delivered by experts based on the studies completed.

Module 22 of computer-aided information processing is engineered to enable the implementation of the function of provision of pharmacological data and the implementation of real-time comparison and analysis of data on various pharmacological groups of medicinal drugs between themselves. In this module, database 25 with details of pharmacological products comprises submodule 30 with details of pharmacological action of drugs (medical reference guides - diagnosis - pathogenesis), submodule 31 with indications (medical reference books [-] clinical symptoms), which is linked [to] the module, submodule 32 with details of drug administration (text from medical reference guides), submodule 33 with details of dosage (text and figures from medical reference guides), submodule 34 with details of side effects, and submodule 35 with details of contraindications. Database 26 with details of drug compatibility and interaction comprises submodule 36, which returns details of pharmacological products incompatible with a given product, submodule 37, which returns details of pharmacological products suppressing one of the actions of the drug, and submodule 38, which returns details of products one of the actions of the drug.

Therefore, when entering the name of a medicinal drug, the attending physician sees on the display of the computerized workstation other and trade names of this drug and receives information on its pharmacological action, indication, use, and details of dosages, side effects and contraindications. In this context, the attending physician gets a full picture of the options for using this medicinal drug in the treatment option picked by him in terms of its compatibility with other drugs chosen by him for the treatment (details of pharmacological products incompatible with this drug, of pharmacological products suppressing or boosting one of the actions of the drug).

When determining in module 9 the treatment method or option, the attending physician receives information about the tactics of this treatment and, accordingly, receives information from database 25, which will make it possible to finalize the set of medicinal drugs in accordance with the patient's condition.

### Industrial Applicability

This invention may find application in the industry and will make it possible to improve the effectiveness of the process of making a decision that is reliable for the patient and will ensure optimal treatment.

## Claims

1. A clinical information system **characterized in that** it contains the computerized workstations of an attending physician, the workstations being equipped with input/output devices and linked by a network with a communication controller with a subsystem for the input of source data of each patient, the subsystem of which comprises:
- a module for the input of the patient's basic details,
- a module for the input of information from the medical history with the patient's complaints,
- a module for the input of details of the clinical tests done by the attending physician on the patient,
- a module for the input of information based on the results of instrumentation and laboratory tests,
- a module for the input of details of treatment options and methods, and
- a module for the recording of diagnosis made by the attending physician, wherein for enabling the differentiation of clinical, instrumentation and laboratory data by the attending physician in order to make a diagnosis, the subsystem for the input of source data is linked by a data bus with an information support subsystem, which is engineered with functionality for displaying information and reference material on the monitor of the computerized workstation of the attending physician consistent with the format of the data entered into the subsystem for input of source data of each patient and which comprises:
- - a module with information on general reference sources, which is linked to the module for the input of the patient's basic details,
- - a module with information on the groups of diseases that contain matches for the patient's complaints, which [module] is linked to the module for the input of information from the medical history with the patient's complaints,
- - a module with information on clinical tests, which is linked to the module for the input of details of clinical tests,
- - a module with information on instrumentation and laboratory tests,
which is linked to the module for the input of information based on the results of instrumentation and laboratory tests,
- -a module with information on the forms of standards of generic and specific treatment, which is linked to the module for the input of details of treatment options and methods, and
- -a module with information on the diagnoses made, which is linked to the module for the recording of the diagnosis made by the attending physician, wherein the subsystem for the input of source data being linked via the common bus with the common medical database, the module of smart analysis and statistical processing of information, the module of computer-aided information processing, which modules are interlinked in the data exchange mode, and the medical reporting module, the module of smart analysis and statistical processing of information being engineered to enable the implementation of the function of mathematical and analytical treatment of the patient's details in real time based on the particulars of the patient's clinical condition, dynamics of disease progress and response to the treatment, wherein the module of computer-aided information processing being engineered to enable the implementation of the function of provision of pharmacological data and the implementation of real-time comparison and analysis of data on various pharmacological groups of medicinal drugs between themselves, and comprises data bus-interlinked database on pharmacological products and a database on drug compatibility and interaction.
